# EUROPEAN PATENT APPLICATION

(11) **EP 4 470 626 A1**
(43) Date of publication of application: **04.12.2024**
(21) Application number: 23747118.0
(22) Date of filing: 27.01.2023
(51) Int. Cl.: A61Q 1/14, A61Q 19/10, A61K 8/03, A61K 8/31, A61K 8/33, A61K 8/34, A61K 8/44

(54) **TWO-LAYER-SEPARATED CLEANSING AGENT COMPOSITION**

(30) Priority: 28.01.2022 JP 2022012423
(71) Applicant: AJINOMOTO CO., INC., Chuo-ku Tokyo 104-8315 (JP)
(72) Inventor: IKEDA, Naoaki, Kawasaki-shi, Kanagawa 210-8681 (JP)
(74) Representative: Mewburn Ellis LLP
(86) International application number: PCT/JP2023/002748
(87) International publication number: WO 2023/145908

(57) **Abstract**

The present invention relates to a two-layer separated cleansing composition consisting of an aqueous layer containing (a) N-acyl acidic amino acid or a salt thereof, (b) a cationic surfactant, and (c) alcohol having not more than 6 carbon atoms, and an oil layer containing (e) a liquid oil agent, wherein the component (a) is (a1) a mixture of N-acyl acidic amino acid having an acyl group having 8 to 18 carbon atoms or a salt thereof, the component (c) includes (c1) monovalent alcohol having not more than 6 carbon atoms, and (c2) polyhydric alcohol having not more than 6 carbon atoms, or the component (a) includes two or more kinds of the aforementioned component (a1), or the aforementioned component (a1) and (a2) N-acyl acidic amino acid having an acyl group having 12 to 16 carbon atoms or a salt thereof, and component (c) is the aforementioned component (c2).

According to the present invention, a two-layer separated cleansing composition which is separated into two layers of an aqueous layer and an oil layer, is superior in the washability of makeup cosmetics and the like, the feeling of use, and separativeness into two layers after use, has foamability, and is improved in the usability can be provided.

## Description

### [Technical Field]

The present invention relates to a two-layer separated cleansing composition which is separated into two layers of an aqueous layer and an oil layer, and superior in foamability.

### [Background Art]

Cleansing compositions that are primarily used to remove makeup cosmetics are broadly classified into water-type, oil-type, and emulsion-type, based on the characteristics of the blended components therein.

Water types contain surfactants, alcohol, and water as the main components, and are popular because of a refreshing feel of use after washing. However, due to the absence of oil agents, they are inferior in cleansing performance.

Oil type and emulsion type can contain oil agents at high contents, but they may cause strong stickiness after rinsing, thus requiring double cleansing with a different facial cleanser after cleansing in some cases. To solve this problem, formulations that mix an aqueous phase with a large amount of oil phase have been considered. Generally, when an aqueous phase is mixed with a large amount of oil phase, it is difficult to maintain a uniform emulsified state. It has thus been proposed to deliberately separate the aqueous phase and oil phase into two layers, thereby simultaneously achieving an aesthetic appearance and stability of the cleansing composition.

For example, a technique for obtaining cosmetics suitable for removing makeup cosmetics and cleanup of the skin, including forming a two-layer cosmetic that is emulsified by shaking and separated into two layers by standing still, by blending an N-long chain acyl basic amino acid derivative or an acid addition salt thereof such as N-coconut oil fatty acid acyl-L-arginine ethyl ester DL-pyrrolidonecarboxylic acid salt, into a two-layer cosmetic containing an oil agent and water (Patent Literature 1) and, in a two-layer cleansing cosmetic consisting of a liquid aqueous layer containing benzalkonium chloride or N-coconut oil fatty acid acyl-L-arginine ethyl ester DL-pyrrolidonecarboxylic acid salt, and a liquid oil layer, wherein the aforementioned aqueous layer further contains an amphoteric surfactant and polyhydric alcohol, and the aforementioned oil layer contains a liquid oil agent, a technique for not only maintaining the storage stability of the formulation, but also maintaining a two-layer system, as well as achieving uniform mixing when in use and maintaining the uniform state for a certain period of time (Patent Literature 2) are disclosed.

However, when removing makeup cosmetics, the area around the eyes needs to be washed, and the eyes are mostly closed during washing. Thus, conventional cleansing compositions have problems in that affinity of the cleansing composition for makeup cosmetics and the end point of the washing action are difficult to notice. The techniques proposed in the above-mentioned Patent Literatures 1 and 2 do not refer to improvements in usability, such as making the affinity of the cleansing composition for makeup cosmetics and the end point of the washing action easily noticed.

Therefore, a two-layer cleansing composition that has a good aesthetic appearance, is superior in the washability of makeup cosmetics and the like and the feeling of use, and improved in the usability such as affinity for makeup cosmetics and ease of seeing the end point of the washing action has been desired.

### [Citation List]

### [Patent Literature]

[Patent Literature 1]
   JP-A-2004-196713
[Patent Literature 2]
   JP-A-2010-222321

### [Summary of Invention]

### [Technical Problem]

Therefore, the present invention aims to provide a two-layer separated cleansing composition which is separated into two layers of an aqueous layer and an oil layer, is superior in the washability of makeup cosmetics and the like, the feeling of use, and separativeness into two layers after use, has foamability, and is improved in the usability such as affinity for makeup cosmetics and ease of seeing the end point of the washing action.

### [Solution to Problem]

The present inventor has conducted intensive studies in an attempt to solve the above-mentioned problems and found that a two-layer separated cleansing composition consisting of an aqueous layer containing (a) N-acyl acidic amino acid or a salt thereof, (b) a cationic surfactant, and (c) alcohol having not more than 6 carbon atoms, and an oil layer containing (e) a liquid oil agent, wherein the component (a) includes (a1) a mixture of N-acyl acidic amino acid having an acyl group having 8 to 18 carbon atoms or a salt thereof, and the component (c) includes (c1) monovalent alcohol having not more than 6 carbon atoms, and (c2) polyhydric alcohol having not more than 6 carbon atoms, or the component (a) includes two or more kinds of (a1) a mixture of N-acyl acidic amino acid having an acyl group having 8 to 18 carbon atoms or a salt thereof, and the component (c) includes (c2) polyhydric alcohol having not more than 6 carbon atoms, or the component (a) includes (a1) a mixture of N-acyl acidic amino acid having an acyl group having 8 to 18 carbon atoms or a salt thereof and (a2) N-acyl acidic amino acid having an acyl group having 12 to 16 carbon atoms or a salt thereof, and the component (c) includes (c2) polyhydric alcohol having not more than 6 carbon atoms, can solve the above-mentioned problems. He has conducted further studies and completed the present invention.

The present inventor has also found that a composition containing (a1) a mixture of N-acyl acidic amino acid having an acyl group having 8 to 18 carbon atoms or a salt thereof as component (a), (c2) polyhydric alcohol having not more than 6 carbon atoms as component (c), and N-acyl neutral amino acid having an acyl group having 8 to 18 carbon atoms or a salt thereof further as component (f) can solve the above-mentioned problems.

That is, the present invention relates to the following.
[1] A two-layer separated cleansing composition consisting of an aqueous layer comprising (a) N-acyl acidic amino acid or a salt thereof, (b) a cationic surfactant, and (c) an alcohol having not more than 6 carbon atoms, and an oil layer comprising (e) a liquid oil agent, wherein the (a) N-acyl acidic amino acid or a salt thereof includes (a1) a mixture of N-acyl acidic amino acid having an acyl group having 8 to 18 carbon atoms or a salt thereof, and the (c) alcohol having not more than 6 carbon atoms includes (c1) monovalent alcohol having not more than 6 carbon atoms, and (c2) polyhydric alcohol having not more than 6 carbon atoms.
[2] A two-layer separated cleansing composition consisting of an aqueous layer comprising (a) an N-acyl acidic amino acid or a salt thereof, (b) a cationic surfactant, and (c) an alcohol having not more than 6 carbon atoms, and an oil layer comprising (e) a liquid oil agent, wherein the (a) N-acyl acidic amino acid or a salt thereof includes two or more kinds of (a1) a mixture of N-acyl acidic amino acid having an acyl group having 8 to 18 carbon atoms or a salt thereof, and the (c) alcohol having not more than 6 carbon atoms includes (c2) polyhydric alcohol having not more than 6 carbon atoms.
[3] A two-layer separated cleansing composition consisting of an aqueous layer comprising (a) N-acyl acidic amino acid or a salt thereof, (b) a cationic surfactant, and (c) an alcohol having not more than 6 carbon atoms, and an oil layer comprising (e) a liquid oil agent, wherein the (a) N-acyl acidic amino acid or a salt thereof includes (a1) a mixture of N-acyl acidic amino acid having an acyl group having 8 to 18 carbon atoms or a salt thereof and (a2) N-acyl acidic amino acid having an acyl group having 12 to 16 carbon atoms or a salt thereof, and the (c) alcohol having not more than 6 carbon atoms includes (c2) polyhydric alcohol having not more than 6 carbon atoms.
[4] The cleansing composition of any of [1] to [3], wherein the (a1) mixture of the N-acyl acidic amino acid having an acyl group having 8 to 18 carbon atoms or a salt thereof is a mixture of the N-acyl glutamic acid having an acyl group having 8 to 18 carbon atoms or a salt thereof.
[5] The cleansing composition of [3] or [4], wherein the (a2) N-acyl acidic amino acid with an acyl group having 12 to 16 carbon atoms or a salt thereof is N-acyl glutamic acid with an acyl group having 12 to 16 carbon atoms or a salt thereof.
[6] The cleansing composition of any of [1] to [5], wherein the (b) cationic surfactant is an alkyl ester of N-acyl basic amino acid or an acid addition salt thereof.
[7] The cleansing composition of [6], wherein the alkyl ester of N-acyl basic amino acid or an acid addition salt thereof is an alkyl ester having 1 to 3 carbon atoms of N-acyl basic amino acid with an acyl group having 8 to 18 carbon atoms or an acid addition salt thereof.
[8] The cleansing composition of any of [1], [4], [6], and [7], wherein the (c1) monovalent alcohol having not more than 6 carbon atoms is ethanol.
[9] The cleansing composition of any of [1] to [8], wherein the (c2) polyhydric alcohol having not more than 6 carbon atoms is at least one kind selected from the group consisting of 1,3-butyleneglycol, glycerol, and sorbitol.
[10] The cleansing composition of any of [1] to [9], wherein a content of the (c) alcohol having not more than 6 carbon atoms is 0.1 mass % to 8 mass %.
[11] The cleansing composition of any of [1] to [10], wherein the aqueous layer further comprises (d) basic amino acid or a salt thereof.
[12] The cleansing composition of [11], wherein the (d) basic amino acid or a salt thereof is arginine or a salt thereof.
[13] The cleansing composition of any of [1] to [12], wherein the (e) liquid oil agent is at least one kind selected from the group consisting of light isoparaffin and liquid ether oil.
[14] The cleansing composition of any of [1] to [13], wherein a content ratio of the aqueous layer and the oil layer is 10:90 to 90:10 in a mass ratio.
[15] The cleansing composition of any of [1] to [14], wherein the viscosity at 25°C is not more than 100 mPa·s.
[16] A two-layer separated cleansing composition consisting of an aqueous layer comprising (a) N-acyl acidic amino acid or a salt thereof, (b) a cationic surfactant, and (c) an alcohol having not more than 6 carbon atoms, and an oil layer comprising (e) a liquid oil agent, wherein the (a) N-acyl acidic amino acid or a salt thereof includes (a1) a mixture of N-acyl acidic amino acid having an acyl group having 8 to 18 carbon atoms or a salt thereof, the (c) alcohol having not more than 6 carbon atoms includes (c2) polyhydric alcohol having not more than 6 carbon atoms, and the aqueous layer comprises (f) N-acyl neutral amino acid having an acyl group having 8 to 18 carbon atoms or a salt thereof.
[17] The cleansing composition of [16], wherein the (a1) mixture of the N-acyl acidic amino acid having an acyl group having 8 to 18 carbon atoms or a salt thereof is a mixture of the N-acyl glutamic acid having an acyl group having 8 to 18 carbon atoms or a salt thereof.
[18] The cleansing composition of [16] or [17], wherein the (b) cationic surfactant is an alkyl ester of N-acyl basic amino acid or an acid addition salt thereof.
[19] The cleansing composition of [18], wherein the alkyl ester of N-acyl basic amino acid or an acid addition salt thereof is an alkyl ester having 1 to 3 carbon atoms of N-acyl basic amino acid having an acyl group having 8 to 18 carbon atoms or an acid addition salt thereof.
[20] The cleansing composition of any of [16] to [19], wherein the (c2) polyhydric alcohol having not more than 6 carbon atoms is at least one kind selected from the group consisting of 1,3-butyleneglycol, glycerol, and sorbitol.
[21] The cleansing composition of any of [16] to [20], wherein a content of the (c) alcohol having not more than 6 carbon atoms is 0.1 mass % to 8 mass %.
[22] The cleansing composition of any of [16] to [21], wherein the aqueous layer further comprises (d) basic amino acid or a salt thereof.
[23] The cleansing composition of [22], wherein the (d) basic amino acid or a salt thereof is arginine or a salt thereof.
[24] The cleansing composition of any of [16] to [23], wherein the (e) liquid oil agent is at least one kind selected from the group consisting of light isoparaffin and liquid ether oil.
[25] The cleansing composition of any of [16] to [24], wherein the (f) N-acyl neutral amino acid having an acyl group having 8 to 18 carbon atoms or a salt thereof is N-acyl glycine having an acyl group having 8 to 18 carbon atoms or a salt thereof.
[26] The cleansing composition of any of [16] to [25], wherein a content ratio of the aqueous layer and the oil layer is 10:90 to 90:10 in a mass ratio.
[27] The cleansing composition of any of [16] to [26], wherein the viscosity at 25°C is not more than 100 mPa·s.

### [Advantageous Effects of Invention]

According to the present invention, a two-layer separated cleansing composition can be provided, which has a beautiful appearance of an aqueous layer and an oil layer separated into two layers, is emulsified when in use by shaking to mix well the aqueous layer and the oil layer, and can be well separated again into the aqueous layer and the oil layer or the emulsion layer and the oil layer after use, by allowing them to stand for a certain period of time.

The two-layer separated cleansing composition of the present invention exhibits good washability for makeup cosmetics and affords a refreshing feeling of use.

Furthermore, the two-layer separated cleansing composition of the present invention exhibits good foamability, and can clearly indicate the appropriate end-point of the washing action and rinsing by the presence of foam.

### [Description of Embodiments]

The two-layer separated cleansing composition of the present invention (hereinafter also to be referred to as "the cleansing composition of the present invention" in the present specification) consists of an aqueous layer containing (a) N-acyl acidic amino acid or a salt thereof, (b) a cationic surfactant, and (c) an alcohol having not more than 6 carbon atoms, and an oil layer comprising (e) a liquid oil agent.

In the first embodiment, the cleansing composition of the present invention contains (a1) a mixture of N-acyl acidic amino acid having an acyl group having 8 to 18 carbon atoms or a salt thereof as (a) N-acyl acidic amino acid or a salt thereof, and (c1) monovalent alcohol having not more than 6 carbon atoms and (c2) polyhydric alcohol having not more than 6 carbon atoms as (c) alcohol having not more than 6 carbon atoms.

In the second embodiment, the cleansing composition of the present invention contains two or more kinds of (a1) mixtures of N-acyl acidic amino acid having an acyl group having 8 to 18 carbon atoms or a salt thereof as (a) N-acyl acidic amino acid or a salt thereof, and (c2) polyhydric alcohol having not more than 6 carbon atoms as (c) alcohol having not more than 6 carbon atoms.

Furthermore, in the third embodiment, the cleansing composition of the present invention contains (a1) a mixture of N-acyl acidic amino acid having an acyl group having 8 to 18 carbon atoms or a salt thereof and (a2) N-acyl acidic amino acid having an acyl group having 12 to 16 carbon atoms or a salt thereof as (a) N-acyl acidic amino acid or a salt thereof and (c2) polyhydric alcohol having not more than 6 carbon atoms as (c) alcohol having not more than 6 carbon atoms.

The N-acyl acidic amino acid contained as component (a) in the aqueous layer of the cleansing composition of the present invention is an N-acylated derivative of acidic amino acid. The acidic amino acid of the derivative is, for example, an amino acid showing acidity such as glutamic acid, aspartic acid, 2-aminoadipic acid, cysteine acid, homocysteine acid and the like, and may be any of L-form, D-form, and DL-form. L-form and DL-form are preferably used and L-form is more preferably used.

For the purpose of the present invention, N-acyl glutamic acid and N-acyl aspartic acid are preferably used, and N-acyl glutamic acid is more preferably used as the N-acyl acidic amino acid.

Examples of the acyl group of the above-mentioned N-acyl acidic amino acid include a straight chain or branched chain saturated acyl group having about 8 to 18 carbon atoms such as octanoyl (capryloyl), 6-methylheptanoyl (isooctanoyl), 2-ethylhexanoyl, decanoyl (caprinoyl), dodecanoyl (lauroyl), tetradecanoyl (myristoyl), 12-methyltridecanoyl (isomyristoyl), hexadecanoyl (palmitoyl), 14-methylpentadecanoyl (isopalmitoyl), octadecanoyl (stearoyl), 16-methylheptadecanoyl (isostearoyl), and the like, and a straight chain or branched chain unsaturated acyl group having about 8 to 18 carbon atoms such as octenoyl, 6-methylheptenoyl (isooctenoyl), decenoyl, dodecenoyl, tetradecenoyl, 9-hexadecenoyl (palmitoleoyl), 9-octadecenoyl (oleoyl), and the like. It may be a mixture of a saturated acyl group and an unsaturated acyl group, each having about 8 to 18 carbon atoms, such as coconut oil fatty acid acyl (cocoyl), palm oil fatty acid acyl, and the like.

As a salt of the above-mentioned N-acyl acidic amino acid, alkali metal salts such as sodium salt, potassium salt and the like; alkaline earth metal salts such as magnesium salt, calcium salt and the like; ammonium salt; organic amine salts such as diethanolamine salt, triethanolamine salt and the like; basic amino acid salts such as lysine salt, arginine salt and the like; and the like can be mentioned and, from the aspects of easy availability and handling property, sodium salt, potassium salt or triethanolamine salt is preferred, and sodium salt or potassium salt is particularly preferred.

As (a1) a mixture of the N-acyl acidic amino acid having an acyl group having 8 to 18 carbon atoms or a salt thereof contained as component (a) in the two-layer separated cleansing composition of the first embodiment of the present invention (hereinafter also to be referred to as "the cleansing composition of the first embodiment of the present invention" in the present specification), a mixture of N-acyl acidic amino acid having a saturated acyl group and an unsaturated acyl group having about 8 to 18 carbon atoms such as coconut oil fatty acid acyl (cocoyl), palm oil fatty acid acyl, and the like, or a salt thereof is preferably used.

Examples of the mixture of such N-acyl acidic amino acid or a salt thereof include N-coconut oil fatty acid acyl aspartic acid, sodium N-coconut oil fatty acid acyl aspartate, disodium N-coconut oil fatty acid acyl aspartate, potassium N-coconut oil fatty acid acyl aspartate, triethanolamine N-coconut oil fatty acid acyl aspartate, N-coconut oil fatty acid acyl glutamic acid, sodium N-coconut oil fatty acid acyl glutamate, disodium N-coconut oil fatty acid acyl glutamate, potassium N-coconut oil fatty acid acyl glutamate, triethanolamine N-coconut oil fatty acid acyl glutamate, N-palm oil fatty acid acyl aspartic acid, sodium N-palm oil fatty acid acyl aspartate, disodium N-palm oil fatty acid acyl aspartate, potassium N-palm oil fatty acid acyl aspartate, triethanolamine N-palm oil fatty acid acyl aspartate, N-palm oil fatty acid acyl glutamic acid, sodium N-palm oil fatty acid acyl glutamate, disodium N-palm oil fatty acid acyl glutamate, potassium N-palm oil fatty acid acyl glutamate, triethanolamine N-palm oil fatty acid acyl glutamate and the like, and sodium N-coconut oil fatty acid acyl glutamate, disodium N-coconut oil fatty acid acyl glutamate, potassium N-coconut oil fatty acid acyl glutamate, sodium N-palm oil fatty acid acyl glutamate, disodium N-palm oil fatty acid acyl glutamate, potassium N-palm oil fatty acid acyl glutamate and the like are more preferably used.

In the present invention, a mixture of N-acyl acidic amino acid having an acyl group having 8 to 18 carbon atoms or a salt thereof may be synthesized by a method known per se, for example, by a Schotten-Baumann reaction of acidic amino acid, and fatty acid halide converted from fatty acid by a conventional method and the like under alkali conditions. Alternatively, commercially available products provided by Ajinomoto Co., Inc. and others for cosmetics or skin cleanser can also be used.

In the present invention, as a mixture of the N-acyl acidic amino acid having an acyl group having 8 to 18 carbon atoms or a salt thereof, a mixture of one kind may be used, or two or more kinds of mixtures different in the composition of acyl group and the kind of acidic amino acid can also be used in combination.

Two or more kinds of mixtures of (a1) N-acyl acidic amino acid having an acyl group having 8 to 18 carbon atoms or a salt thereof which are different in the composition of acyl group are preferably used as component (a) because the two-layer separativeness of the cleansing composition is improved.

As component (a), the below-mentioned component (a2) can further be used in combination in addition to the above-mentioned component (a1). Such case is preferred because the two-layer separativeness of the cleansing composition is improved.

The (a1) mixture of N-acyl acidic amino acid having an acyl group having 8 to 18 carbon atoms or a salt thereof contained as component (a) in the two-layer separated cleansing composition in the second embodiment of the present invention (hereinafter also to be referred to as "the cleansing composition of the second embodiment of the present invention" in the present specification) is as described above as regards the cleansing composition of the first embodiment of the present invention.

In the cleansing composition of the second embodiment of the present invention, two or more kinds of (a1) mixtures of N-acyl acidic amino acid or a salt thereof which are different in the composition of acyl group are preferably combined as component (a1). It is more preferable to combine an acidic amino acid having an acyl group which is induced from fatty acid obtained from coconut oil or a salt thereof with an acidic amino acid having an acyl group which is induced from fatty acid obtained from palm oil or a salt thereof, and it is further preferable to combine N-coconut oil fatty acid acyl glutamic acid salt with N-palm oil fatty acid acyl glutamic acid salt. As the N-coconut oil fatty acid acyl glutamic acid salt, sodium N-coconut oil fatty acid acyl glutamate is preferably used, and as the N-palm oil fatty acid acyl glutamic acid salt, sodium N-palm oil fatty acid acyl glutamate is preferably used.

The (a1) mixture of N-acyl acidic amino acid having an acyl group having 8 to 18 carbon atoms or a salt thereof contained as component (a) in the two-layer separated cleansing composition in the third embodiment of the present invention (hereinafter also to be referred to as "the cleansing composition of the third embodiment of the present invention" in the present specification) is as described above as regards the cleansing composition of the first embodiment of the present invention.

In the cleansing composition of the third embodiment of the present invention, examples of the (a2) N-acyl acidic amino acid having an acyl group having 12 to 16 carbon atoms or a salt thereof which is contained together with (a1) a mixture of the N-acyl acidic amino acid having an acyl group having 8 to 18 carbon atoms or a salt thereof as component (a) include N-lauroyl aspartic acid, sodium N-lauroyl aspartate, disodium N-lauroyl aspartate, potassium N-lauroyl aspartate, triethanolamine N-lauroyl aspartate, N-myristoyl aspartic acid, sodium N-myristoyl aspartate, disodium N-myristoyl aspartate, potassium N-myristoyl aspartate, triethanolamine N-myristoyl aspartate, N-palmitoyl aspartic acid, sodium N-palmitoyl aspartate, disodium N-palmitoyl aspartate, potassium N-palmitoyl aspartate, triethanolamine N-palmitoyl aspartate, N-lauroyl glutamic acid, sodium N-lauroyl glutamate, disodium N-lauroyl glutamate, potassium N-lauroyl glutamate, triethanolamine N-lauroyl glutamate, N-myristoyl glutamic acid, sodium N-myristoyl glutamate, disodium N-myristoyl glutamate, potassium N-myristoyl glutamate, triethanolamine N-myristoyl glutamate, N-palmitoyl glutamic acid, sodium N-palmitoyl glutamate, disodium N-palmitoyl glutamate, potassium N-palmitoyl glutamate, triethanolamine N-palmitoyl glutamate and the like. Among these, sodium N-lauroyl glutamate, disodium N-lauroyl glutamate, potassium N-lauroyl glutamate, sodium N-myristoyl glutamate, disodium N-myristoyl glutamate, potassium N-myristoyl glutamate, sodium N-palmitoyl glutamate, disodium N-palmitoyl glutamate, potassium N-palmitoyl glutamate, and the like are more preferably used.

In the cleansing composition of the third embodiment of the present invention, the above-mentioned N-acyl acidic amino acid having an acyl group having 12 to 16 carbon atoms or a salt thereof may be synthesized by a method known per se, for example, by a Schotten-Baumann reaction of acidic amino acid, and fatty acid halide converted from fatty acid by a conventional method and the like under alkali conditions. Alternatively, commercially available products provided by Ajinomoto Co., Inc. and others for cosmetics or skin cleanser can also be used.

One kind of the N-acyl acidic amino acid having an acyl group having 12 to 16 carbon atoms or a salt thereof may be selected and used alone, or two or more kinds thereof may be selected and used in combination.

The content of (a) N-acyl acidic amino acid or a salt thereof in the cleansing composition of the present invention, that is, the content of component (a1) in the cleansing composition of the first embodiment and the cleansing composition of the second embodiment of the present invention, and a total content of component (a1) and component (a2) in the cleansing composition of the first embodiment of the present invention containing component (a2) in addition to component (a1) as component (a), and the cleansing composition of the third embodiment of the present invention, is each preferably 0.5 mass % to 10 mass %, more preferably 1 mass % to 8 mass %.

In the cleansing composition of the present invention, as the cationic surfactant contained in the aqueous layer as component (b), an N-acyl basic amino acid derivative or an acid addition salt thereof is preferably used.

As the N-acyl basic amino acid derivative used in the present invention, an alkyl ester of N-acylated basic amino acid can be mentioned. The basic amino acid in the derivative may be, for example, lysine, arginine, histidine, ornithine, citrulline, or the like, and may be any of L-form, D-form, and DL-form. L-form and DL-form are preferably used and L-form is more preferably used.

As the alkyl ester of N-acyl basic amino acid, alkyl esters having about 1 to 3 carbon atoms such as methyl ester, ethyl ester, propyl ester, and the like can be mentioned.

For the purpose of the present invention, as the N-acyl basic amino acid derivative, N-acyl arginine methyl ester, N-acyl arginine ethyl ester, N-acyl lysine methyl ester, N-acyl lysine ethyl ester, N-acyl ornithine methyl ester, N-acyl ornithine ethyl ester, and the like are preferably used, and N-acyl arginine ethyl ester is more preferably used.

Examples of the acyl group of the above-mentioned N-acyl basic amino acid derivative include a straight chain or branched chain saturated acyl group having about 6 to 22 carbon atoms such as hexanoyl (caproyl), octanoyl (capryloyl), 6-methylheptanoyl (isooctanoyl), 2-ethylhexanoyl, decanoyl (caprinoyl), dodecanoyl (lauroyl), tetradecanoyl (myristoyl), 12-methyltridecanoyl (isomyristoyl), hexadecanoyl (palmitoyl), 14-methylpentadecanoyl (isopalmitoyl), octadecanoyl (stearoyl), 16-methylheptadecanoyl (isostearoyl), eicosanoyl (arachidoyl), docosanoyl (behenoyl), and the like, and a straight chain or branched chain unsaturated acyl group having about 6 to 22 carbon atoms such as hexsenoyl, octenoyl, 6-methylheptenoyl (isooctenoyl), decenoyl, dodecenoyl, tetradecenoyl, 9-hexadecenoyl (palmitoleoyl), 9-octadecenoyl (oleoyl), docosenoyl, and the like. It may be a mixture of a saturated acyl group and an unsaturated acyl group, each having about 8 to 18 carbon atoms, such as coconut oil fatty acid acyl (cocoyl), palm oil fatty acid acyl, and the like.

For the purpose of the present invention, a straight chain or branched chain, saturated or unsaturated acyl group having about 8 to 18 carbon atoms, and a mixture of a saturated acyl group and an unsaturated acyl group, each having about 8 to 18 carbon atoms, are preferred, and a straight chain saturated acyl group having about 8 to 18 carbons and coconut oil fatty acid acyl(cocoyl) are more preferred.

Examples of the acid addition salts with the above-mentioned N-acyl basic amino acid derivative include salts with inorganic acids and salts with organic acids.

Examples of the salts with inorganic acids include salts with hydrohalic acid (hydrochloric acid, hydrobromic acid, hydroiodic acid, etc.), sulfuric acid, nitric acid, phosphoric acid, and the like.

Examples of the salts with organic acids include salts with monocarboxylic acids such as formic acid, acetic acid, propanoic acid, and the like; salts with saturated dicarboxylic acids such as oxalic acid, malonic acid, succinic acid, and the like; salts with unsaturated dicarboxylic acids such as maleic acid, fumaric acid, and the like; salts with tricarboxylic acids such as citric acid and the like; salts with keto acids such as α-ketoglutaric acid and the like; salts with amino acids forming lactam such as pyrrolidonecarboxylic acid and the like; salts with organic sulfonic acids such as methanesulfonic acid (mesylic acid), p-toluenesulfonic acid, and the like; and the like.

From the aspects of obtainability and handleability, hydrochloride and pyrrolidonecarboxylic acid salt are preferred.

In the present invention, N-acyl basic amino acid derivatives and acid addition salts thereof can be used without particular limitation as long as they can be appropriately used in cosmetics or cleansing compositions for skin. N-coconut oil fatty acid acyl-L-arginine ethyl ester DL-pyrrolidonecarboxylic acid salt and N-lauroyl-L-arginine ethyl ester hydrochloride are more preferably used, and N-coconut oil fatty acid acyl-L-arginine ethyl ester DL-pyrrolidonecarboxylic acid salt is particularly preferably used.

In the present invention, the N-acyl basic amino acid derivative and acid addition salts thereof may be synthesized by a method known per se. Alternatively, commercially available products provided by Ajinomoto Co., Inc. and others for cosmetics or skin cleansers can also be used.

In the present invention, one kind of the N-acyl basic amino acid described above or a salt thereof may be selected and used alone, or two or more kinds thereof may be selected and used in combination.

The content of the (b) N-acyl basic amino acid or an acid addition salt thereof in the cleansing composition of the present invention is preferably 0.05 mass % to 5 mass %, more preferably 0.1 mass % to 3 mass %.

The cleansing composition of the first embodiment of the present invention contains (c1) monovalent alcohol having not more than 6 carbon atoms and (c2) polyhydric alcohol having not more than 6 carbon atoms as component (c).

In addition, the cleansing compositions of the second embodiment and the third embodiment of the present invention contains (c2) polyhydric alcohol having not more than 6 carbon atoms as component (c).

The (c1) monovalent alcohol having not more than 6 carbon atoms which is contained as component (c) in the cleansing composition of the first embodiment of the present invention can be used without particular limitation as long as it can be appropriately used in cosmetics or cleansing compositions for skin. For example, ethanol, propanol, isopropanol, butanol, pentanol, hexanol, and the like can be mentioned, among which ethanol can be preferably used.

The (c2) polyhydric alcohol having not more than 6 carbon atoms which is contained as component (c) in the cleansing compositions of the first embodiment, the second embodiment, and the third embodiment of the present invention can be used without particular limitation as long as it can be appropriately used in cosmetics or cleansing compositions for skin. Examples thereof include divalent alcohols such as propylene glycol (1,2-propanediol), 1,3-propanediol, dipropylene glycol, 1,3-butyleneglycol, 1,2-pentanediol, 1,2-hexanediol and the like; trivalent alcohols such as glycerol and the like; tetritols such as erythritol, threitol and the like; pentitols such as arabinitol, xylitol, ribitol and the like; hexitols such as iditol, galactitol, sorbitol, mannitol and the like; and the like, and 1,3-butyleneglycol, glycerol, sorbitol, and the like can be preferably used.

One kind each of the above-mentioned monovalent alcohol having not more than 6 carbon atoms and polyhydric alcohol having not more than 6 carbon atoms can be selected and used alone, or two or more kinds thereof can be selected and used in combination.

In the present invention, as the monovalent alcohol having not more than 6 carbon atoms and the polyhydric alcohol having not more than 6 carbon atoms, commercially available products provided by various companies for use in cosmetics or skin cleansers can be used.

The content of (c1) monovalent alcohol having not more than 6 carbon atoms in the cleansing composition of the first embodiment of the present invention is preferably 0.1 mass % to 5 mass %, more preferably 0.5 mass % to 3 mass %.

The content of (c2) polyhydric alcohol having not more than 6 carbon atoms in the cleansing compositions of the first embodiment, the second embodiment, and the third embodiment, of the present invention is preferably less than 10 mass %, more preferably 0.5 mass % to 6 mass %.

The content of (c) alcohol having not more than 6 carbon atoms in the cleansing composition of the present invention, that is, a total content of component (c1) and component (c2) in the cleansing composition of the first embodiment of the present invention, and the content of component (c2) in the cleansing compositions of the second embodiment and the third embodiment of the present invention is preferably 0.1 mass % to 8 mass %, more preferably 0.5 mass % to 8 mass %, from the aspect of the foamability of the cleansing composition of the present invention.

In the cleansing composition in each embodiment of the first embodiment to the third embodiment of the present invention, from the aspect of foam sustainability, (d) basic amino acid or a salt thereof is preferably further contained in the aqueous layer in addition to the above-mentioned components (a) to (c).

As the basic amino acid that can be contained as component (d) in the cleansing composition of the present invention, lysine, arginine, histidine, ornithine, citrulline and the like can be mentioned, and lysine, arginine, and histidine are preferably used and arginine is more preferably used. As such basic amino acid, any of L-form, D-form, and DL-form is used, L-form and DL-form are preferably used, and L-form is more preferably used.

The salt of the above-mentioned basic amino acid is not particularly limited as long as it is a pharmaceutically acceptable salt appropriately used in cosmetics or cleansing compositions for skin, and acid addition salts, salts with bases, and the like can be mentioned.

Specifically, salts with inorganic bases, organic bases, inorganic acids, and organic acids, salts with amino acids, and the like can be mentioned.

Examples of the salts with inorganic bases include salts with alkali metals such as lithium, sodium, potassium and the like, salts with alkaline earth metals such as magnesium, calcium and the like, ammonium salt, and the like.

Examples of the salts with organic bases include salts with alkanol amines such as monoethanolamine, diethanolamine, triethanolamine and the like, salts with heterocyclic amines such as morpholine, piperidine and the like, and the like.

Examples of the salts with inorganic acids include salts with hydrohalic acid (hydrochloric acid, hydrobromic acid, hydroiodic acid, etc.), sulfuric acid, nitric acid, phosphoric acid, and the like, and the like.

Examples of the salts with organic acids include salts with monocarboxylic acids such as formic acid, acetic acid, propanoic acid, and the like; salts with saturated dicarboxylic acids such as oxalic acid, malonic acid, succinic acid, and the like; salts with unsaturated dicarboxylic acids such as maleic acid, fumaric acid, and the like; salts with tricarboxylic acids such as citric acid and the like; salts with keto acids such as α-ketoglutaric acid and the like; and the like.

Examples of the salts with amino acid include salts with aliphatic amino acids such as alanine and the like; salts with aromatic amino acids such as tyrosine and the like; salts with acidic amino acids such as aspartic acid, glutamic acid and the like; salts with amino acids forming lactam such as pyrrolidonecarboxylic acid and the like; and the like.

The above-mentioned salts may each be hydrate (hydrate salt), and examples of such hydrate include 1 hydrate - 6 hydrate and the like.

In the present invention, free forms and hydrochlorides are preferably used as the basic amino acid or a salt thereof.

The above-mentioned basic amino acid and a salt thereof to be used may be extracted and purified from naturally occurring animals and plants, or may be obtained by chemical synthesis, fermentation methods, enzymatic methods, or gene recombination methods. Alternatively, commercially available products provided by various companies for use in cosmetics or skin cleanser can also be used.

In the cleansing composition of the present invention, one kind of the above-mentioned basic amino acid or a salt thereof may be used alone, or two or more kinds thereof may be selected and used in combination.

The content of the (d) basic amino acid or a salt thereof in the cleansing composition of the present invention is preferably 0.01 mass % to 1 mass %, more preferably 0.05 mass % to 0.5 mass %.

When component (d) is contained in the form of a salt, the content is expressed as the amount converted to a free form.

In the cleansing composition of the present invention, from the aspect of foamability, the content ratio of component (a) and component (b) [(a):(b)], that is, the content ratio of component (a1) and component (b) [(a1):(b)] in the cleansing composition of the first embodiment of the present invention and the cleansing composition of the second embodiment of the present invention, and the content ratio of a total of component (a1) and component (a2), and component (b) [{(a1)+(a2)}:(b)] in the cleansing composition of the first embodiment of the present invention containing component (a2) in addition to component (a1) and the cleansing composition of the third embodiment of the present invention, is preferably 1:0.05 to 1:0.8, more preferably 1:0.1 to 1:0.7, in a mass ratio.

Also, from the aspect of foamability, the content ratio of component (a) and component (c) [(a):(c)], that is, the content ratio of component (a1) and a total of component (c1) and component (c2) [(a1):{(c1)+(c2)}] in the cleansing composition of the first embodiment of the present invention, the content ratio of a total of component (a1) and component (a2) and a total of component (c1) and component (c2) [{(a1)+(a2)}:{(c1)+(c2)}] in the cleansing composition of the first embodiment of the present invention containing component (a2) in addition to component (a1) , the content ratio of component (a1) and component (c2) [(a1): (c2)] in the cleansing composition of the second embodiment of the present invention, and the content ratio of a total of component (a1) and component (a2), and component (c2) [{(a1)+(a2)1:(c2)] in the cleansing composition of the third embodiment of the present invention, is preferably 1:0.8 to 1:5, more preferably 1:0.8 to 1:4, in a mass ratio.

Furthermore, from the aspect of foamability, the content ratio of component (a) and component (d) [(a):(d)], that is, the content ratio of component (a1) and component (d) [(a1):(d)] in the cleansing composition of the first embodiment of the present invention and the cleansing composition of the second embodiment of the present invention, and the content ratio of a total of component (a1) and component (a2), and component (d) [{(a1)+(a2)}:(d)] in the cleansing composition of the first embodiment of the present invention containing component (a2) in addition to component (a1) and the cleansing composition of the third embodiment of the present invention, is preferably 1:0.05 to 1:0.15, more preferably 1:0.05 to 1:0.12, in a mass ratio.

The cleansing composition of each embodiment of the first embodiment to the third embodiment of the present invention contains (e) a liquid oil agent in the oil layer in order to improve removability of makeup cosmetics.

The liquid oil agent that can be contained as component (e) in the oil layer in the cleansing composition of the present invention is not particularly limited as long as it is liquid at room temperature and can be appropriately contained in cosmetics or cleansing compositions for skin, and liquid hydrocarbon oil, liquid ether oil, liquid ester oil, liquid fatty acid, liquid animal and vegetable oils, liquid wax, liquid silicone oil, and the like can be mentioned.

In the present specification, the "room temperature" means room temperature as defined in the General Rules of the Japanese Pharmacopoeia, 18th Edition, namely, 1°C to 30°C.

Examples of the liquid hydrocarbon oil include α-olefin oligomer, light isoparaffin (isododecane, isohexadecane, etc.), light liquid paraffin (hydrogenated polyisobutene, etc.), liquid paraffin (hydrogenated polyisobutene, etc.), liquid paraffin (mineral oil), squalane, plant-derived squalene, and the like.

Examples of the liquid ether oil include dicaprylyl ether and the like.

Examples of the liquid ester oil include 2-ethylhexanoic acid cetyl, isononanoic acid isononyl, myristic acid isopropyl, myristic acid octyldodecyl, myristic acid isostearyl, palmitic acid isopropyl, palmitic acid 2-ethylhexyl, sebacic acid dibutyloctyl, malic acid diisostearyl, dicapric acid propylene glycol, monocaprylic acid propylene glycol, dicaprylic acid propylene glycol, diisostearic acid propylene glycol, tri(caprylic · capric acid) glyceryl, tri(2-ethylhexanoic acid) glyceryl ester and the like.

Examples of the liquid fatty acid include palmitoleic acid, isostearic acid, oleic acid, linoleic acid, linolenic acid, and the like.

Examples of the liquid animal and vegetable oils include avocado oil, almond oil, apricot kernel oil, olive oil, safflower oil, corn oil, sunflower oil, grape seed oil, hazelnut oil, coconut oil, whale oil, and the like.

Examples of the liquid wax include jojoba oil and the like.

Examples of the liquid silicone oil include chain silicones such as methylpolysiloxane, methylhydrogen polysiloxane, methylphenylpolysiloxane and the like; cyclic silicones such as octamethylcyclotetrasiloxane, decamethylcyclopentasiloxane, dodecamethylcyclohexasiloxane and the like; amino-modified silicones such as aminoethylaminopropylmethylsiloxane · dimethylsiloxane copolymer, aminopropylmethylsiloxane · dimethylsiloxane copolymer and the like; polyether-modified silicones such as polyoxyethylene · methylpolysiloxane copolymer and the like; and the like.

From the aspects of the removability of makeup cosmetics and the effect on the feeling of use, the cleansing composition of the present invention preferably uses esters of lower alcohols and fatty acids such as myristic acid isopropyl and the like, triglycerides such as tri(2-ethylhexanoic acid) glyceryl ester and the like, light isoparaffins such as isododecane, isohexadecane and the like, liquid ether oils such as dicaprylyl ether, and the like, and light isoparaffins such as isododecane, isohexadecane and the like, liquid ether oils such as dicaprylyl ether and the like, and the like are particularly preferably used.

As the liquid oil agents mentioned above, commercially available products provided by various companies for use in cosmetics or skin cleansers can be used.

In the cleansing composition of the present invention, one kind of the above-mentioned liquid oil agents may be used alone, or two or more kinds thereof may be selected and used in combination.

The content of the component (e) in the cleansing composition of the present invention is preferably 10 mass % to 90 mass %, more preferably 10 mass % to 60 mass %.

In the cleansing composition of the present invention, water-soluble or hydrophilic general additives can be added to the aqueous layer as long as the characteristics of the present invention are not impaired.

Examples of such additive include suspending agents such as propylene glycol alginate, dioctyl sodium sulfosuccinate, soybean lecithin, povidone and the like; thickening agents such as carboxyvinyl polymer, sodium polyacrylate, xanthan gum, carboxymethylcellulose, hydroxypropylcellulose, poly(vinyl alcohol) (partially hydrolyzed) and the like; pH adjusters such as hydrochloric acid, phosphoric acid, succinic acid, sodium hydroxide, potassium hydroxide, sodium acetate, sodium hydrogenphosphate, 1-amino-2-propanol, citric acid, trisodium citrate, lactic acid and the like; stabilizers such as disodium ethylenediaminetetraacetate, polyoxyethylene polyoxypropylene glycol and the like; antioxidants such as ascorbic acid, sodium erythorbate and the like; antiseptics such as sorbic acid, sodium dehydroacetate, methyl p-hydroxybenzoate, phenoxyethanol and the like; and the like. Where necessary, one or more kinds thereof can be used.

In the cleansing composition of the present invention, oil-soluble or lipophilic general additives can be added to the oil layer as long as the characteristics of the present invention are not impaired.

Examples of such additive include antioxidants such as tocopheryl acetate, dibutyl hydroxytoluene, γ-oryzanol and the like; antiseptics such as propyl p-hydroxybenzoate, butyl p-hydroxybenzoate and the like; flavors such as geraniol, citral, limonene, lavender oil and the like; ultraviolet absorbers such as methoxycinnamic acid 2-ethylhexyl, salicylic acid 2-ethylhexyl, 2-hydroxy-4-methoxybenzophenone, 2-cyano-3,3-diphenylprop-2-enoic acid 2-ethylhexyl, 2-[4-(diethylamino)-2-hydroxybenzoyl]benzoic acid hexyl and the like; and the like. One or more kinds thereof can be used according to the purpose.

The ratio of the aqueous layer and the oil layer (aqueous layer:oil layer) in the cleansing composition of the present invention is preferably 10:90 to 90:10, more preferably 15:85 to 85:15, in a mass ratio.

The cleansing composition of the first embodiment of the present invention can be produced as follows. First, component (a1) or component (a1) and component (a2) as component (a), component (b), component (c1) and component (c2) as component (c), or the aforementioned components and component (d) are added to water together with water-soluble or hydrophilic general additives as necessary, and mixed to prepare a homogeneous aqueous layer. On the other hand, component (e) and oil-soluble or lipophilic general additives as necessary are mixed to prepare a homogeneous oil layer. Then, the aforementioned oil layer is added to the aforementioned aqueous layer to give a two-layer separated cleansing composition.

The cleansing composition of the second embodiment of the present invention can be produced as follows. First, component (a1) as component (a), component (b), component (c2) as component (c), or the aforementioned components and component (d) are added to water together with water-soluble or hydrophilic general additives as necessary, and mixed to prepare a homogeneous aqueous layer. On the other hand, component (e) and oil-soluble or lipophilic general additives as necessary are mixed to prepare a homogeneous oil layer. Then, the aforementioned oil layer is added to the aforementioned aqueous layer to give a two-layer separated cleansing composition.

The cleansing composition of the third embodiment of the present invention can be produced as follows. First, component (a1) and component (a2) as component (a), component (b), component (c2) as component (c), or the aforementioned components and component (d) are added to water together with water-soluble or hydrophilic general additives as necessary, and mixed to prepare a homogeneous aqueous layer. On the other hand, component (e) and oil-soluble or lipophilic general additives as necessary are mixed to prepare a homogeneous oil layer. Then, the aforementioned oil layer is added to the aforementioned aqueous layer to give a two-layer separated cleansing composition.

As water, water suitable for the production of cosmetics or cleansing compositions for skin, for example, purified water such as distilled water, ion exchange water, and the like can be used.

The content of water in the cleansing composition of the present invention is set as an amount with respect to the total amount of the cleansing composition of the present invention as 100 mass %.

The cleansing composition of the present invention has a beautiful appearance of an aqueous layer and an oil layer separated into two layers, is emulsified when in use by shaking to mix well the aqueous layer and the oil layer, and can be well separated again into the aqueous layer and the oil layer after use, by allowing them to stand for a certain period of time.

Also, the cleansing composition of the present invention exhibits good washability for makeup cosmetics and affords a refreshing feeling of use. Furthermore, the cleansing composition of the present invention exhibits good foamability, and clearly indicates the appropriate end-point of the washing action and rinsing by the presence of foam.

Since the cleansing composition of the present invention shows good foamability, it can be provided by filling in a foamer container.

The foamer container is a non-gas type foam discharge container in which one or more porous bodies with a given pore size is/are used, a cleansing composition and air are mixed in a predetermined ratio, and the mixture is discharged in the form of foam.

As the non-gas type foam discharge container, a squeeze foamer container that discharges foam by manually squeezing the bottle barrel part, a pump foamer container that discharges foam by pushing down the nozzle, and the like can be mentioned. A container made from plastic such as high-density polyethylene, polypropylene, polyethylene terephthalate, or the like is preferably used.

As such foamer container, containers manufactured by Daiwa Can Co., Ltd., Yoshino Kogyosho Co., Ltd., and the like can be used.

As the porous body for forming foams in a foamer container, a plastic net or sponge made of nylon, polyester (polyethylene terephthalate, etc.), polypropylene, and the like can be used. The pore size of the porous body is generally 25 um to 300 µm, preferably 50 um to 200 µm.

When the cleansing composition of the present invention is filled in a foamer container and provided, from the aspect of foam ejection from the foamer container, the viscosity of the cleansing composition of the present invention is preferably not more than 100mPa·s, more preferably not more than 20mPa·s, at 25°C. The viscosity of the cleansing composition of the present invention is generally not less than 1 mpa·s at 25°C.

The viscosity of the cleansing composition of the present invention can be calculated from the measured value after rotating for 30 seconds at 25°C with rotor No. 1 at a rotation speed of 30 rpm using a Brookfield viscometer ("TVB-10" (Toki Sangyo Co., Ltd.), etc.).

The cleansing composition of the present invention can be preferably provided as cosmetics for skin washing, particularly, as cleansing cosmetics for removing makeup cosmetics.

Furthermore, the present invention provides, as the fourth embodiment, a two-layer separated cleansing composition consisting of an aqueous layer containing (a1) a mixture of the N-acyl acidic amino acid having an acyl group having 8 to 18 carbon atoms or a salt thereof as (a) N-acyl acidic amino acid or a salt thereof, (b) a cationic surfactant, (c2) polyhydric alcohol having not more than 6 carbon atoms as (c) alcohol having not more than 6 carbon atoms, and (f) N-acyl neutral amino acid having an acyl group having 8 to 18 carbon atoms or a salt thereof, and an oil layer containing (e) a liquid oil agent.

The (a1) mixture of N-acyl acidic amino acid having an acyl group having 8 to 18 carbon atoms or a salt thereof contained as component (a) in the two-layer separated cleansing composition in the fourth embodiment of the present invention (hereinafter also to be referred to as "the cleansing composition of the fourth embodiment of the present invention" in the present specification) is as described above as regards the cleansing composition of the first embodiment of the present invention.

The (b) cationic surfactant, (c2) polyhydric alcohol having not more than 6 carbon atoms contained as (c) alcohol having not more than 6 carbon atoms, and (e) a liquid oil agent, contained in the cleansing composition of the fourth embodiment of the present invention, are as described above as regards the cleansing compositions of the first embodiment to the third embodiment of the present invention.

In the cleansing composition of the fourth embodiment of the present invention, the content of each of component (a) (i.e., component (a1)), component (b), component (c) (i.e., component (c2)) and component (e), the content ratio of component (a) and component (b) (i.e., [(a1):(b)]), and the content ratio of component (a) and component (c) (i.e., [(a1):(c2)]) are also as described above as regards the cleansing compositions of the first embodiment to the third embodiment of the present invention.

The N-acyl neutral amino acid contained as component (f) in the aqueous layer of the fourth embodiment of the cleansing composition of the present invention is an N-acylated derivative of neutral amino acid. The neutral amino acid of the derivative is, for example, an amino acid showing neutrality such as glycine, methylglycine (sarcosine), alanine, methylalanine and the like. When it contains an optical isomer, it may be any of L-form, D-form, and DL-form. L-form and DL-form are preferably used, and L-form is more preferably used.

For the purpose of the present invention, N-acyl glycine and N-acyl alanine are preferably used as N-acyl neutral amino acid, and N-acyl glycine is more preferably used.

The acyl group in the N-acyl neutral amino acid is an acyl group having 8 to 18 carbon atoms, and such acyl group is the same as the acyl group in the N-acyl acidic amino acid.

As a salt of N-acyl neutral amino acid, alkali metal salts such as sodium salt, potassium salt and the like; alkaline earth metal salts such as magnesium salt, calcium salt and the like; ammonium salt; organic amine salts such as diethanolamine salt, triethanolamine salt and the like; and the like can be mentioned and, from the aspects of easy availability and handling property, sodium salt, potassium salt or triethanolamine salt is preferable, and sodium salt or potassium salt is particularly preferred.

Examples of the N-acyl neutral amino acid or a salt thereof preferably used in the cleansing composition of the fourth embodiment of the present invention include N-coconut oil fatty acid acyl glycine, sodium N-coconut oil fatty acid acyl glycinate, potassium N-coconut oil fatty acid acyl glycinate, N-palm oil fatty acid acyl alanine, sodium N-palm oil fatty acid acyl alaninate, potassium N-palm oil fatty acid acyl alaninate, and the like.

In the present invention, N-acyl neutral amino acid having an acyl group having 8 to 18 carbon atoms or a salt thereof may be synthesized by a method known per se, for example, by a Schotten-Baumann reaction of neutral amino acid, and fatty acid halide converted from fatty acid by a conventional method and the like under alkali conditions. Alternatively, commercially available products provided by Ajinomoto Co., Inc. and others, for cosmetics or skin cleanser can also be used.

In the present invention, one kind of N-acyl neutral amino acid having an acyl group having 8 to 18 carbon atoms or a salt thereof may be used, or two or more kinds thereof can also be used in combination.

The content of component (f) in the cleansing composition of the fourth embodiment of the present invention is preferably 1 mass % to 6 mass %, more preferably 1.5 mass % to 4.5 mass %.

Also in the cleansing composition of the fourth embodiment of the present invention, from the aspect of foam sustainability, (d) basic amino acid or a salt thereof is preferably further contained in the aqueous layer in addition to the above-mentioned component (a1), component (b), component (c) and component (f).

The (d) basic amino acid or a salt thereof contained in the cleansing composition of the fourth embodiment of the present invention, the content of component (d), and the content ratio of component (a) and component (d) (i.e., [(a1):(d)]) are as described above as regards the cleansing compositions of the first embodiment to the third embodiment of the present invention.

Also in the cleansing composition of the fourth embodiment of the present invention, similar to the cleansing compositions of the first embodiment to the third embodiment of the present invention, water-soluble or hydrophilic general additives and oil-soluble or lipophilic general additives can be added to the aqueous layer and the oil layer respectively as long as the characteristics of the present invention are not impaired.

In the cleansing composition of the fourth embodiment of the present invention, fatty acid salts such as coconut oil fatty acid potassium salt, coconut oil fatty acid sodium salt and the like can be added to the aqueous layer together with N-acyl neutral amino acid having an acyl group having 8 to 18 carbon atoms or a salt thereof which is component (f).

The ratio of the aqueous layer and the oil layer (aqueous layer:oil layer) in the cleansing composition of the fourth embodiment of the present invention is the same as that in the case of the cleansing compositions of the first embodiment to the third embodiment in the present invention.

The cleansing composition of the fourth embodiment of the present invention can be produced as follows. First, component (a1) as component (a), component (b), component (c2) as component (c), and component (f), or the aforementioned components and component (d) are added to water together with water-soluble or hydrophilic general additives as necessary, and mixed to prepare a homogeneous aqueous layer. On the other hand, component (e) and oil-soluble or lipophilic general additives as necessary are mixed to prepare a homogeneous oil layer. Then, the aforementioned oil layer is added to the aforementioned aqueous layer to give a two-layer separated cleansing composition.

Water contained in the cleansing composition of the fourth embodiment of the present invention and the content thereof are the same as those in the case of the cleansing compositions of the first embodiment to the third embodiment in the present invention.

The cleansing composition of the fourth embodiment of the present invention has an appearance of an aqueous layer and an oil layer separated into two layers, is emulsified when in use by shaking to mix well the aqueous layer and the oil layer, and separated into the emulsion layer and the oil layer after use, by allowing them to stand for a certain period of time.

Also, the cleansing composition of the fourth embodiment of the present invention exhibits good washability for makeup cosmetics and affords a refreshing feeling of use. The cleansing composition of the fourth embodiment of the present invention exhibits better foamability, and more clearly indicates the appropriate end-point of the washing action and rinsing by the presence of foam.

The cleansing composition of the fourth embodiment of the present invention can be provided by filling in a foamer container, similar to the cleansing compositions of the first embodiment to the third embodiment.

The viscosity of the cleansing composition suitable to be filled in a foamer container and provided is as described above as regards the first embodiment to the third embodiment of the cleansing composition.

The cleansing composition of the fourth embodiment of the present invention can be preferably provided as cosmetics for skin washing, particularly, as cleansing cosmetics for removing makeup cosmetics, similar to the cleansing compositions of the first embodiment to the third embodiment.

### [Example]

The present invention is further explained in detail by Examples.

Examples of the cleansing composition of the first embodiment of the present invention are shown below.

### [Examples 1 to 3, Comparative Examples 1 to 5] Two-layer separated cleansing cosmetics

As shown below, as the cleansing composition of the first embodiment of the present invention, two-layer separated cleansing cosmetics of Examples 1 to 3 were prepared, and the two-layer separated cleansing cosmetics of Comparative Examples 1 to 5 were prepared.

In Table 1, components (a1), (a'), (b), (c1), and (c2) were added to ion exchange water, mixed, and homogenized to prepare an aqueous layer. Then, component (e) was added as an oil layer to the aforementioned aqueous layer.

As component (a1), "Amisoft (registered trademark) CS-22" (solid content=25 mass %) (Ajinomoto Co., Inc.) was diluted with water to give an aqueous solution with a solid content of 20 mass %, which was then adjusted with citric acid or sodium hydroxide to the pH shown in Table 1 and used. As the component (a'), "Sunsoft No. 760C" (Taiyo Kagaku Co., Ltd.) was used, as component (b), "CAE (registered trademark)" (Ajinomoto Co., Inc.) was used, as component (c1), "ethanol" (Junsei Chemical Co., Ltd.) was used, and as component (c2), "1,3-BG UK" (Daicel Corporation) and "Sorbitol UK" (Kao Corporation) were used.

For component (e), as dicaprylyl ether, "Cosmacol OE" (Sasol Japan Co., Ltd.) was used, as liquid paraffin (average molecular weight=300), "Moresco White P-55" (MORESCO Corporation) was used, as liquid paraffin (average molecular weight=323), "Moresco White P-70" (MORESCO Corporation) was used, as isododecane, "Marukasol R" (Maruzen Petrochemical Co., Ltd.) was used, and as isohexadecane, "Alamol HD-LQ" (Croda Japan Co., Ltd.) was used.

### [Experimental Example 1] Evaluation of two-layer separativeness and foamability of two-layer separated cleansing cosmetics

The two-layer separated cleansing cosmetics of Examples 1 to 3 and Comparative Examples 1 to 5 were evaluated as follows for the two-layer separativeness and foamability. The evaluation results are also shown in Table 1.

### (1) Preparation of sample

The aqueous layer components were weighed into a 50 mL Falcon tube, heated to 60°C, stirred with a vortex mixer for uniform dissolution, and then the oil layer components were added to prepare each sample of the Examples and Comparative Examples. In addition, to facilitate visual confirmation of the two-layer separativeness, 1 or 2 drops of a 1% by mass aqueous solution of Blue No. 1 were added to the aqueous layer.

### (2) Evaluation of two-layer separativeness

Each sample prepared as described above was shaken up and down vigorously 20 times by hand, then allowed to stand, and visually evaluated for two-layer separativeness according to the following evaluation criteria.

### <Evaluation criteria>

AA: separated neatly into two layers within 12 hr
A: separated neatly into two layers within one day
B: separated neatly into two layers within 2-3 days
C: not separated even after 3 days or more

### (3) Evaluation of foamability

Each sample prepared as described above was shaken up and down vigorously 20 times by hand, then allowed to stand, and the amount of foam after 10 seconds was read from the scale on the Falcon tube. The difference between the total amount of foam and liquid and the amount of liquid drained was taken as the amount of foam, and foamability was visually evaluated according to the following evaluation criteria.

### <Evaluation criteria>

A: foam volume is not less than 40 mL and less than 50 mL
B: foam volume is not less than 35 mL and less than 40 mL
C: foam volume is not less than 30 mL and less than 35 mL
D: foam volume is less than 30 mL

**[Table 1]**

| component | | | | Comp. Ex. 1 | Comp. Ex. 2 | Comp. Ex. 3 | Comp. Ex. 4 | Comp. Ex. 5 | Ex. 1 | Ex. 2 | Ex. 3 |
|---|---|---|---|---|---|---|---|---|---|---|---|
| | (a1) | sodium N-coconut oil fatty acid acyl-L-glutamate (20 mass %, adjusted to pH 7.0) | | | | 5.00 | | 5.00 | | | |
| | | sodium N-coconut oil fatty acid acyl-L-glutamate (20 mass %, adjusted to pH 7.2) | | 5.00 | 5.00 | | | | | | |
| | | sodium N-coconut oil fatty acid acyl-L-glutamate (20 mass %, adjusted to pH 7.6) | | | | | 13.50 | | 13.50 | | |
| | | sodium N-coconut oil fatty acid acyl-L-glutamate (20 mass %, adjusted to pH 7.8) | | | | | | | | 13.50 | 8.00 |
| aqueous layer | | (solid content of sodium N-coconut oil fatty acid acyl-L-glutamate) | | 1.00 | 1.00 | 1.00 | 2.70 | 1.00 | 2.70 | 2.70 | 1.60 |
| | (a') | glyceryl monocaprate | | | | | | 0.50 | | | |
| | (b) | N-coconut oil fatty acid acyl-L-arginine ethyl ester·DL-pyrrolidonecarboxylic acid salt | | | | | 0.30 | | 0.30 | 0.30 | 1.00 |
| | (c1) | ethanol | | | | 3.00 | | | 2.00 | 2.00 | 2.00 |
| | (c2) | 1,3-butyleneglycol | | 10.00 | 6.00 | 10.00 | 3.00 | 10.00 | 3.00 | 3.00 | |
| | | sorbitol | | | | | | | | 3.00 | 4.00 |
| | | ion exchange water | | 45.00 | 39.00 | 32.00 | 53.20 | 34.50 | 51.20 | 48.20 | 55.00 |
| oil layer | (e) | dicaprylyl ether | | | | | 21.00 | | 21.00 | | 21.00 |
| | | liquid paraffin (average molecular weight=300) | | 40.00 | 50.00 | | | | | | |
| | | liquid paraffin (average molecular weight=323) | | | | | | 50.00 | | | |
| | | isododecane | | | | 50.00 | | | | | |
| | | isohexadecane | | | | | 9.00 | | 9.00 | 30.00 | 9.00 |
| total | | | | 100 | 100 | 100 | 100 | 100 | 100 | 100 | 100 |
| (b)/(a) | | | | 0 | 0 | 0 | 0.11 | 0 | 0.11 | 0.11 | 0.63 |
| (c)/(a) | | | | 10.00 | 6.00 | 13.00 | 1.11 | 10.00 | 1.85 | 2.96 | 3.75 |
| aqueous layer/oil layer | | | | 60/40 | 50/50 | 50/50 | 70/30 | 50/50 | 70/30 | 70/30 | 70/30 |
| evaluation item | | | two-layer separativeness | B | C | A | A | A | A | A | A |
| | | | foamability | D | D | D | D | D | B | A | A |
| | | | foam volume (mL) | 26.0 | 25.0 | 11.0 | 23.0 | 21.0 | 38.0 | 45.0 | 45.0 |

| | | | | | | | | | | | |
|---|---|---|---|---|---|---|---|---|---|---|---|
| * Numerical value corresponding to each component in Table shows the content (mass %) of the component. Blank shows that the component is not contained. | | | | | | | | | | | |

As shown in Table 1, the two-layer separated cleansing cosmetics of Examples 1 to 3, which are composed of an aqueous layer containing components (a1), (b), (c1), and (c2) and an oil layer (component (e)) exhibited good two-layer separativeness, and obtained good evaluation of not less than "B" for foamability as well.

In contrast, sufficient foamability was not observed in the two-layer separated cleansing cosmetics of Comparative Examples 1 and 2, which do not contain components (b) and (c1) as aqueous layer components, the two-layer separated cleansing cosmetic of Comparative Example 3, which does not contain component (b), the two-layer separated cleansing cosmetic of Comparative Example 4, which does not contain component (c1), and the two-layer separated cleansing cosmetic of Comparative Example 5, which contains component (a') instead of component (b) and does not contain component (c1).

In addition, in the two-layer separated cleansing cosmetic of Comparative Example 2, which does not contain components (b) and (c1) as aqueous layer components and contains 6.00 mass% of component (c2), good two-layer separativeness was not observed.

The results of the above-mentioned Experimental Example 1 suggest that in order to obtain good two-layer separativeness and foamability in the cleansing composition of the first embodiment of the present invention, it is necessary to contain components (b), (c1), and (c2) as aqueous layer components in addition to component (a1).

### [Examples 4, 5] Two-layer separated cleansing cosmetics

As shown below, as the cleansing composition of the first embodiment of the present invention, two-layer separated cleansing cosmetics of Examples 4 and 5 were prepared.

In Table 2, components (a1), (b), (c1), (c2), and (d) were added to ion exchange water, mixed, and homogenized to prepare an aqueous layer. Then, component (e) was added as an oil layer to the aforementioned aqueous layer.

As component (a1), a starting material prepared in the same manner as in the above-mentioned Examples 1 to 3 was used. As component (b), "CAE (registered trademark)" (Ajinomoto Co., Inc.) was used, as component (c1), "ethanol" (Junsei Chemical Co., Ltd.) was used, as component (c2), "1,3-BG UK" (Daicel Corporation) was used, as component (d), "L-arginine C grade" (Ajinomoto Co., Inc.) was used, and as component (e), "Cosmacol OE" (Sasol Japan Co., Ltd.) and "Alamol HD-LQ" (Croda Japan Co., Ltd.) were used.

### [Experimental Example 2] Study of influence of arginine on foamability of two-layer separated cleansing cosmetics

The two-layer separated cleansing cosmetics of Examples 4 and 5 were evaluated for two-layer separativeness and foamability in the same manner as in Experimental Example 1, together with the two-layer separated cleansing cosmetics of Example 1 and Comparative Example 4 prepared as mentioned above. The evaluation results are also shown in Table 2.

**[Table 2]**

| component | | | | Comp. Ex. 4 | Ex. 1 | Ex. 4 | Ex. 5 |
|---|---|---|---|---|---|---|---|
| aqueous layer | (a1) | sodium N-coconut oil fatty acid acyl-L-glutamate (20 mass %, adjusted to pH 7.6) | | 13.50 | 13.50 | 13.50 | 13.50 |
| | | (solid content of sodium N-coconut oil fatty acid acyl-L-glutamate) | | 2.70 | 2.70 | 2.70 | 2.70 |
| | (b) | N-coconut oil fatty acid acyl-L-arginine ethyl ester·DL-pyrrolidonecarboxylic acid salt | | 0.30 | 0.30 | 0.30 | 0.30 |
| | (c1) | ethanol | | | 2.00 | 2.00 | 2.00 |
| | (c2) | 1,3-butyleneglycol | | 3.00 | 3.00 | 3.00 | 3.00 |
| | | ion exchange water | | 53.20 | 51.20 | 51.05 | 50.96 |
| | (d) | L-arginine | | | | 0.15 | 0.24 |
| oil layer | (e) | dicaprylyl ether | | 21.00 | 21.00 | 21.00 | |
| | | isohexadecane | | 9.00 | 9.00 | 9.00 | 30.00 |
| total | | | | 100 | 100 | 100 | 100 |
| (b)/(a) | | | | 0.11 | 0.11 | 0.11 | 0.11 |
| (c)/(a) | | | | 1.11 | 1.85 | 1.85 | 1.85 |
| (d)/(a) | | | | 0 | 0 | 0.056 | 0.089 |
| aqueous layer/oil layer | | | | 70/30 | 70/30 | 70/30 | 70/30 |
| evaluation item | | | two-layer separativeness | A | A | A | A |
| | | | foamability | D | B | A | A |
| | | | foam volume (mL) | 23.0 | 38.0 | 44.0 | 45.0 |

| | | | | | | | |
|---|---|---|---|---|---|---|---|
| * Numerical value corresponding to each component in Table shows the content (mass %) of the component. Blank shows that the component is not contained. | | | | | | | |

As shown in Table 2, the two-layer separated cleansing cosmetics of Examples 4 and 5, which contain component (d) in addition to components (a1), (b), (c1), and (c2) as aqueous layer components, were found to have improved foamability compared to the two-layer separated cleansing cosmetic of Example 1, which does not contain component (d).

The above-mentioned results of Experimental Example 2 suggest that the foamability of the two-layer separated cleansing composition is improved by further containing component (d) in addition to components (a1), (b), (c1), and (c2) in the cleansing composition of the first embodiment of the present invention.

### [Examples 6 to 12] Two-layer separated cleansing cosmetics

As shown below, as the cleansing composition of the first embodiment of the present invention, two-layer separated cleansing cosmetics of Examples 6 to 12 were prepared.

In Table 3, components (a1), (b), (c1) and (c2), or aforementioned components and further component (d) were added to ion exchange water, mixed, and homogenized to prepare an aqueous layer. Then, component (e) was added as an oil layer to the aforementioned aqueous layer.

Among components (a1), a starting material prepared in the same manner as in the above-mentioned Examples 1 to 3 was used for sodium N-coconut oil fatty acid acyl-L-glutamate, as sodium N-palm oil fatty acid acyl-L-glutamate, "Amisoft (registered trademark) GS-11P" (Ajinomoto Co., Inc.) was used. As component (a2), "Amisoft (registered trademark) MS-11" (Ajinomoto Co., Inc.) and "Amisoft (registered trademark) LS-11" (Ajinomoto Co., Inc.) were used. As component (b), "CAE (registered trademark)" (Ajinomoto Co., Inc.) was used, as component (c1), "ethanol" (Junsei Chemical Co., Ltd.) was used, as component (c2), "1,3-BG UK" (Daicel Corporation) was used, as component (d), "L-arginine C grade" (Ajinomoto Co., Inc.) was used, and as component (e), "Cosmacol OE" (Sasol Japan Co., Ltd.) and "Alamol HD-LQ" (Croda Japan Co., Ltd.) were used.

### [Experimental Example 3] Study of influence of component (a1) and component (a2) on two-layer separativeness of two-layer separated cleansing cosmetics

The two-layer separated cleansing cosmetics of Examples 6 to 12 were evaluated for two-layer separativeness and foamability in the same manner as in the above-mentioned Experimental Example 1, together with the two-layer separated cleansing cosmetics of Examples 1 and 5 prepared as mentioned above. The evaluation results are also shown in Table 3.

**[Table 3]**

| component | | | Ex. 1 | Ex. 5 | Ex. 6 | Ex. 7 | Ex. 8 | Ex. 9 | Ex. 10 | Ex. 11 | Ex. 12 |
|---|---|---|---|---|---|---|---|---|---|---|---|
| | (a1) | sodium N-coconut oil fatty acid acyl-L-glutamate (20 mass %, adjusted to pH 7.6) | 13.50 | 13.50 | 12.75 | 19.20 | 25.60 | 12.75 | 12.75 | 12.75 | 12.75 |
| | | (solid content of sodium N-coconut oil fatty acid acyl-L-glutamate) | 2.70 | 2.70 | 2.55 | 3.84 | 5.12 | 2.55 | 2.55 | 2.55 | 2.55 |
| | | sodium N-palm oil fatty acid acyl-L-glutamate | | | 0.20 | 0.30 | 0.40 | 0.20 | | | 0.20 |
| | (a2) | sodium N-myristoyl-L-glutamate | | | | | | | 0.20 | | |
| aqueous layer | | sodium N-lauroyl-L-glutamate | | | | | | | | 0.20 | |
| | (b) | N-coconut oil fatty acid acyl-L-arginine ethyl ester·DL-pyrrolidonecarboxylic acid salt | 0.30 | 0.30 | 0.45 | 0.68 | 0.90 | 0.45 | 0.45 | 0.45 | 0.45 |
| | (c1) | ethanol | 2.00 | 2.00 | 2.00 | 2.00 | 2.00 | 2.00 | 2.00 | 2.00 | 2.00 |
| | (c2) | 1,3-butyleneglycol | 3.00 | 3.00 | 3.00 | 3.00 | 3.00 | 3.00 | 3.00 | 3.00 | 3.00 |
| | | ion exchange water | 51.20 | 50.96 | 51.60 | 44.82 | 38.10 | 51.36 | 51.36 | 51.36 | 51.36 |
| | (d) | L-arginine | | 0.24 | | | | 0.24 | 0.24 | 0.24 | 0.24 |
| oil layer | (e) | dicaprylyl ether | 21.00 | | 21.00 | 21.00 | 21.00 | | | | 21.00 |
| | | isohexadecane | 9.00 | 30.00 | 9.00 | 9.00 | 9.00 | 30.00 | 30.00 | 30.00 | 9.00 |
| total | | | 100 | 100 | 100 | 100 | 100 | 100 | 100 | 100 | 100 |
| (b)/(a) | | | 0.11 | 0.11 | 0.16 | 0.16 | 0.16 | 0.16 | 0.16 | 0.16 | 0.16 |
| (c)/(a) | | | 1.85 | 1.85 | 1.82 | 1.21 | 0.91 | 1.82 | 1.82 | 1.82 | 1.82 |
| (d)/(a) | | | 0 | 0.089 | 0 | 0 | 0 | 0.087 | 0.087 | 0.087 | 0.087 |
| aqueous layer/oil layer | | | 70/30 | 70/30 | 70/30 | 70/30 | 70/30 | 70/30 | 70/30 | 70/30 | 70/30 |
| evaluation item | | two-layer separativeness | A | A | AA | AA | AA | AA | AA | AA | AA |
| | | foamability | B | A | A | A | A | A | A | A | A |
| | | foam volume (mL) | 38.0 | 45.0 | 41.5 | 45.0 | 42.5 | 40.5 | 45.0 | 45.0 | 45.0 |

| | | | | | | | | | | | |
|---|---|---|---|---|---|---|---|---|---|---|---|
| * Numerical value corresponding to each component in Table shows the content (mass %) of the component. Blank shows that the component is not contained. | | | | | | | | | | | |

As shown in Table 3, the two-layer separated cleansing cosmetics of Examples 6 to 8, which contain two kinds of components (a1) as the component (a), namely, sodium N-coconut oil fatty acid acyl-L-glutamate and sodium N-palm oil fatty acid acyl-L-glutamate, were found to have improved two-layer separativeness as compared to the two-layer separated cleansing cosmetic of Example 1.

Furthermore, the two-layer separated cleansing cosmetics of Examples 9 and 12, which contain two kinds of components (a1) namely, sodium N-coconut oil fatty acid acyl-L-glutamate and sodium N-palm oil fatty acid acyl-L-glutamate, and the two-layer separated cleansing cosmetics of Examples 10 and 11, which contain, in addition to the component (a1), sodium N-myristoyl-L-glutamate or sodium N-lauroyl-L-glutamate as component (a2) were found to have improved two-layer separativeness as compared to the two-layer separated cleansing cosmetic of Example 5.

The results of the above-mentioned Experimental Example 3 suggest that, in the cleansing composition of the first embodiment of the present invention, the two-layer separativeness of the two-layer separated cleansing composition is improved by containing two kinds of components (a1) with different acyl group compositions as component (a), or by containing component (a1) and component (a2).

Examples of the cleansing composition of the second embodiment and the cleansing composition of the third embodiment of the present invention are shown below.

### [Examples 13 to 18, Comparative Examples 6, 7] Two-layer separated cleansing cosmetics

As shown below, as the cleansing composition of the second embodiment of the present invention, the two-layer separated cleansing cosmetic of Example 13 was prepared.

In Table 4, components (a1), (b), and (c2) and other components were added to ion exchange water, mixed, and homogenized to prepare an aqueous layer. Then, component (e) was added as an oil layer to the aforementioned aqueous layer.

As shown below, as the cleansing composition of the third embodiment of the present invention, the two-layer separated cleansing cosmetics of Examples 14 to 18 were prepared.

In Table 4, components (a1), (a2), (b), and (c2) and other components were added to ion exchange water, mixed, and homogenized to prepare an aqueous layer. Then, component (e) was added as an oil layer to the aforementioned aqueous layer.

Furthermore, as shown below, the two-layer separated cleansing cosmetics of Comparative Examples 6, 7 were prepared.

In Table 4, components (a1), (a'), (b), and (c2) and other components were added to ion exchange water, mixed, and homogenized to prepare an aqueous layer. Then, component (e) was added as an oil layer to the aforementioned aqueous layer.

As component (a1), "Amisoft (registered trade mark) CS-22" (solid content =25 mass %) (Ajinomoto Co., Inc.) and "Amisoft (registered trademark) GS-11P" (Ajinomoto Co., Inc.) were used, and as component (a2), "Amisoft (registered trademark) MS-11" (Ajinomoto Co., Inc.) and "Amisoft (registered trademark) LS-11" (Ajinomoto Co., Inc.) were used. As component (a'), "Amisoft (registered trademark) HS-11P" (Ajinomoto Co., Inc.) and "EMAL E-27C" (solid content =27 mass %) (Kao Corporation) were used. As component (b), "CAE (registered trademark)" (Ajinomoto Co., Inc.) and "Everguard LAE-20" (Sino Lion Co., Ltd.) were used, as component (c2), "1,3-BG UK" (Daicel Corporation) was used, and as component (e), "Cosmacol OE" (Sasol Japan Co., Ltd.) and "Alamol HD-LQ" (Croda Japan Co., Ltd.) were used. As other components, trisodium citrate (Junsei Chemical Co., Ltd.) and "sodium hydroxide" (Junsei Chemical Co., Ltd.) were used.

### [Experimental Example 4] Evaluation of two-layer separativeness and foamability of two-layer separated cleansing cosmetics

The two-layer separated cleansing cosmetics of Examples 13 to 18 and Comparative Example 6, 7 were each evaluated for two-layer separativeness and foamability in the same manner as in the above-mentioned Experimental Example 1. The evaluation results are also shown in Table 4.

Each of the cleansing cosmetics of Examples 14 and 15 was measured by a Brookfield viscometer ("TVB-10", Toki Sangyo Co., Ltd.) by rotating for 30 seconds at 25°C with rotor No. 1 at a rotation speed of 30 rpm, and the viscosity at 25°C was determined. As a result, the viscosities of the cleansing cosmetics of Examples 14 and 15 at 25°C were 3.7 mPa·s and 3.6 mPa·s, respectively. It was confirmed that the both are low viscosities suitable for provision in a foamer container.

**[Table 4]**

| component | | | | Ex. 13 | Ex. 14 | Ex. 15 | Ex. 16 | Ex. 17 | Ex. 18 | Comp. Ex. 6 | Comp. Ex. 7 |
|---|---|---|---|---|---|---|---|---|---|---|---|
| | (a1) | sodium N-coconut oil fatty acid acyl-L-glutamate (25 mass %, pH unadjusted) | | 12.40 | 12.40 | 12.40 | 12.40 | 12.40 | 12.40 | 12.40 | |
| | | (solid content of sodium N-coconut oil fatty acid acyl-L-qlutamate) | | 3.10 | 3.10 | 3.10 | 3.10 | 3.10 | 3.10 | 3.10 | |
| | | sodium N-palm oil fatty acid acyl-L-qlutamate | | 0.30 | | | | | | | |
| | (a2) | sodium N-myristoyl-L-glutamate | | | 0.20 | 0.10 | 0.30 | 0.20 | | | |
| | | sodium N-laurovl-L-qlutamate | | | 0.20 | 0.30 | 0.10 | 0.20 | 0.30 | | |
| | (a') | sodium N-stearovl-L-qlutamate | | | | | | | | 0.30 | |
| aqueous layer | | sodium polyoxyethylene lauryl ether sulfate (27 mass % aqueous solution) | | | | | | | | | 12.60 (solid content= 3.40) |
| | (b) | N-coconut oil fatty acid acyl-L-arginine ethyl ester DL-pyrrolidonecarboxylic acid salt | | 0.60 | 0.60 | 0.60 | 0.60 | | 0.60 | 0.60 | 0.60 |
| | | 20 mass % glycerol solution of N-lauroyl-L-arginine ethyl ester hydrochloride | | | | | | 3.70 (solid content= 0.74) | | | |
| | (c2) | 1,3-butyleneglycol | | 3.70 | 3.70 | 3.70 | 3.70 | 3.70 | 3.70 | 3.70 | 3.70 |
| | | ion exchange water | | 65.35 | 65.25 | 65.25 | 65.25 | 62.15 | 65.35 | 65.35 | 65.45 |
| | other | trisodium citrate | | 2.45 | 2.45 | 2.45 | 2.45 | 2.45 | 2.45 | 2.45 | 2.45 |
| | | 20 mass % sodium hydroxide aqueous solution | | 0.20 | 0.20 | 0.20 | 0.20 | 0.20 | 0.20 | 0.20 | 0.20 |
| oil layer | (e) | dicaprylyl ether | | 10.50 | 10.50 | 10.50 | 10.50 | 10.50 | 10.50 | 10.50 | 10.50 |
| | | isohexadecane | | 4.50 | 4.50 | 4.50 | 4.50 | 4.50 | 4.50 | 4.50 | 4.50 |
| total | | | | 100 | 100 | 100 | 100 | 100 | 100 | 100 | 100 |
| (b)/(a) | | | | 0.18 | 0.17 | 0.17 | 0.17 | 0.21 | 0.18 | 0.18 | - |
| (c)/(a) | | | | 1.09 | 1.06 | 1.06 | 1.06 | 1.06 | 1.09 | 1.09 | - |
| aqueous layer/oil layer | | | | 85/15 | 85/15 | 85/15 | 85/15 | 85/15 | 85/15 | 85/15 | 85/15 |
| evaluation item | | | two-layer separativeness | B | AA | AA | A | A | A | B | C |
| | | | foamability | A | A | A | B | B | B | D | C |
| | | | foam volume (mL) | 42.5 | 44.0 | 44.0 | 36.0 | 35.0 | 39.0 | 22.5 | 32.5 |

| | | | | | | | | | | | |
|---|---|---|---|---|---|---|---|---|---|---|---|
| * Numerical value corresponding to each component in Table shows the content (mass %) of the component. Blank shows that the component is not contained. ** In Table, "-" means uncalculatable because of the absence of component (a). | | | | | | | | | | | |

As shown in Table 4, good two-layer separativeness and foamability were observed in the two-layer separated cleansing cosmetic of Example 13 of the present invention, which contains two kinds of components (a1) as the component (a) in the aqueous layer together with component (b) and component (c2), and the two-layer separated cleansing cosmetics of Examples 14 to 18 of the present invention, which contain component (a1) and component (a2) in the aqueous layer together with component (b) and component (c2). Particularly, improved two-layer separativeness was observed in addition to good foamability in the two-layer separated cleansing cosmetics of Examples 14 and 15, which contain sodium N-myristoyl-L-glutamate 0.20 mass % and sodium N-lauroyl-L-glutamate 0.20 mass %, or sodium N-myristoyl-L-glutamate 0.10 mass % and sodium N-lauroyl-L-glutamate 0.30 mass %, as component (a2) together with component (a1), and contain N-coconut oil fatty acid acyl-L-arginine ethyl ester·DL-pyrrolidonecarboxylic acid salt as component (b).

On the other hand, sufficient foamability was not observed in the two-layer separated cleansing cosmetic of Comparative Example 6, which contains sodium N-stearoyl-L-glutamate as component (a') instead of component (a2). The two-layer separated cleansing cosmetic of Comparative Example 7, which contains sodium polyoxyethylene lauryl ether sulfate as component (a') instead of component (a1) and component (a2) showed reduced two-layer separativeness and foamability.

The results of the above-mentioned Experimental Example 4 suggest that it is necessary to contain two or more kinds of component (a1), component (b), and component (c2) in the aqueous layer in order to achieve good two-layer separativeness and foamability in the cleansing composition of the second embodiment of the present invention. It was also suggested that component (a1) and component (a2), component (b), and component (c2) need to be contained in the aqueous layer in order to achieve good two-layer separativeness and foamability in the cleansing composition of the third embodiment of the present invention.

Examples of the cleansing composition of the fourth embodiment of the present invention are shown below.

### [Example 19] Two-layer separated cleansing cosmetics

As shown below, as the cleansing composition of the fourth embodiment of the present invention, two-layer separated cleansing cosmetic of Example 19 was prepared.

In Table 5, components (a1), (b), (c2), and (f) and other components were added to ion exchange water, mixed, and homogenized to prepare an aqueous layer. Then, component (e) was added as an oil layer to the aforementioned aqueous layer.

The pH of the two-layer separated cleansing cosmetic of Example 19 was 7.52.

### [Experimental Example 5] Evaluation of two-layer separativeness and foamability of two-layer separated cleansing cosmetics

The two-layer separated cleansing cosmetic of Example 19 was evaluated for two-layer separativeness and foamability in the same manner as in the above-mentioned Experimental Example 1.

The two-layer separated cleansing cosmetic of Example 19 is shaken and then left standing, and separated into two layers of an emulsion layer and an oil layer. In this Experimental Example, the two-layer separativeness was evaluated in terms of separation into an emulsion layer and an oil layer, according to the evaluation criteria in the above-mentioned Experimental Example 1.

The evaluation results are also shown in Table 5.

**[Table 5]**

| component | | | starting material name | supplier | Ex. 19 |
|---|---|---|---|---|---|
| aqueous layer | (a1) | sodium N-coconut oil fatty acid acyl-L-glutamate (25 mass %, pH unadjusted) | AMISOFT (registered trademark) CS-22 | Ajinomoto Co., Inc. | 12.5 (solid content=3.1) |
| | (f) | potassium N-coconut oil fatty acid acyl glycinate (21 mass % aqueous solution) | AMILITE (registered trademark) GCK-12H | Ajinomoto Co., Inc. | 12.5 (solid content=2.6) |
| | (b) | N-coconut oil fatty acid acyl-L-arginine ethyl ester·DL-pyrrolidonecarboxylic acid salt | CAE (registered trademark) | Ajinomoto Co., Inc. | 0.6 |
| | (c2) | 1,3-butyleneglycol | 1,3-BG UK | Daicel Corporation | 3.7 |
| | | ion exchange water | - | - | 53.1 |
| | other | coconut oil fatty acid potassium salt (9 mass % aqueous solution) | AMILITE (registered trademark) GCK-12H | Ajinomoto Co., Inc. | 1.1 (solid content) |
| | | trisodium citrate | trisodium citrate | Junsei Chemical Co., Ltd. | 2.5 |
| | | 20 mass % sodium hydroxide aqueous solution | sodium hydroxide | Junsei Chemical Co., Ltd. | 0.1 |
| oil layer | (e) | dicaprylyl ether | Cosmacol OE | Sasol Japan Co., Ltd. | 5.0 |
| | | isohexadecane | Alamol HD-LQ | Croda Japan Co., Ltd. | 10.0 |
| total | | | | | 100 |
| (b) / (a) | | | | | 0.19 |
| (c) / (a) | | | | | 1.19 |
| aqueous layer/oil layer | | | | | 85/15 |
| evaluation item | | two-layer separativeness | | | AA |
| | | foamability | | | A |
| | | foam volume (mL) | | | >45 |

| | | | | | |
|---|---|---|---|---|---|
| * Numerical value corresponding to each component in Table shows the content (mass %) of the component. ** In Table, coconut oil fatty acid potassium salt is component contained in potassium N-coconut oil fatty acid acyl glycinate aqueous solution. | | | | | |

As shown in Table 5, the two-layer separated cleansing cosmetics of Example 19 exhibited good two-layer separativeness and also very good foamability.

The above-mentioned results of Experimental Example 5 confirm that the cleansing composition of the fourth embodiment of the present invention, which contains (f) N-acyl neutral amino acid having an acyl group having 8 to 18 carbon atoms or a salt thereof in the aqueous layer together with (a1) a mixture of the N-acyl acidic amino acid having an acyl group having 8 to 18 carbon atoms or a salt thereof, (b) a cationic surfactant, (c2) polyhydric alcohol having not more than 6 carbon atoms exhibits good two-layer separativeness into an emulsion layer and an oil layer and is superior in foamability.

### [Industrial Applicability]

As described in detail above, according to the present invention, a two-layer separated cleansing composition can be provided, which has a beautiful appearance of an aqueous layer and an oil layer separated into two layers, is emulsified when in use by shaking to mix well the aqueous layer and the oil layer, and can be well separated again into the aqueous layer and the oil layer or the emulsion layer and the oil layer after use, by allowing them to stand for a certain period of time.

The two-layer separated cleansing composition of the present invention exhibits good washability for makeup cosmetics and affords a refreshing feeling of use.

Furthermore, the two-layer separated cleansing composition of the present invention exhibits good foamability, and can clearly indicate the appropriate end-point of the washing action and rinsing by the presence of foam.

This application is based on a patent application No. 2022-012423 filed in Japan, the contents of which are incorporated in full herein.

## Claims

1. A two-layer separated cleansing composition consisting of an aqueous layer comprising (a) N-acyl acidic amino acid or a salt thereof, (b) a cationic surfactant, and (c) an alcohol having not more than 6 carbon atoms, and an oil layer comprising (e) a liquid oil agent, wherein the (a) N-acyl acidic amino acid or a salt thereof comprises (a1) a mixture of N-acyl acidic amino acid having an acyl group having 8 to 18 carbon atoms or a salt thereof, and the (c) alcohol having not more than 6 carbon atoms comprises (c1) monovalent alcohol having not more than 6 carbon atoms, and (c2) polyhydric alcohol having not more than 6 carbon atoms.

2. A two-layer separated cleansing composition consisting of an aqueous layer comprising (a) an N-acyl acidic amino acid or a salt thereof, (b) a cationic surfactant, and (c) an alcohol having not more than 6 carbon atoms, and an oil layer comprising (e) a liquid oil agent, wherein the (a) N-acyl acidic amino acid or a salt thereof comprises two or more kinds of (a1) a mixture of N-acyl acidic amino acid having an acyl group having 8 to 18 carbon atoms or a salt thereof, and the (c) alcohol having not more than 6 carbon atoms comprises (c2) polyhydric alcohol having not more than 6 carbon atoms.

3. A two-layer separated cleansing composition consisting of an aqueous layer comprising (a) N-acyl acidic amino acid or a salt thereof, (b) a cationic surfactant, and (c) an alcohol having not more than 6 carbon atoms, and an oil layer comprising (e) a liquid oil agent, wherein the (a) N-acyl acidic amino acid or a salt thereof comprises (a1) a mixture of N-acyl acidic amino acid having an acyl group having 8 to 18 carbon atoms or a salt thereof and (a2) N-acyl acidic amino acid having an acyl group having 12 to 16 carbon atoms or a salt thereof, and the (c) alcohol having not more than 6 carbon atoms comprises (c2) polyhydric alcohol having not more than 6 carbon atoms.

4. The cleansing composition according to any one of claims 1 to 3, wherein the (a1) mixture of the N-acyl acidic amino acid having an acyl group having 8 to 18 carbon atoms or a salt thereof is a mixture of the N-acyl glutamic acid having an acyl group having 8 to 18 carbon atoms or a salt thereof.

5. The cleansing composition according to claim 3, wherein the (a2) N-acyl acidic amino acid with an acyl group having 12 to 16 carbon atoms or a salt thereof is N-acyl glutamic acid with an acyl group having 12 to 16 carbon atoms or a salt thereof.

6. The cleansing composition according to any one of claims 1 to 3, wherein the (b) cationic surfactant is an alkyl ester of N-acyl basic amino acid or an acid addition salt thereof.

7. The cleansing composition according to claim 6, wherein the alkyl ester of N-acyl basic amino acid or an acid addition salt thereof is an alkyl ester having 1 to 3 carbon atoms of N-acyl basic amino acid with an acyl group having 8 to 18 carbon atoms or an acid addition salt thereof.

8. The cleansing composition according to claim 1, wherein the (c1) monovalent alcohol having not more than 6 carbon atoms is ethanol.

9. The cleansing composition according to any one of claims 1 to 3, wherein the (c2) polyhydric alcohol having not more than 6 carbon atoms is at least one kind selected from the group consisting of 1,3-butyleneglycol, glycerol, and sorbitol.

10. The cleansing composition according to any one of claims 1 to 3, wherein a content of the (c) alcohol having not more than 6 carbon atoms is 0.1 mass % to 8 mass %.

11. The cleansing composition according to any one of claims 1 to 3, wherein the aqueous layer further comprises (d) basic amino acid or a salt thereof.

12. The cleansing composition according to claim 11, wherein the (d) basic amino acid or a salt thereof is arginine or a salt thereof.

13. The cleansing composition according to any one of claims 1 to 3, wherein the (e) liquid oil agent is at least one kind selected from the group consisting of light isoparaffin and liquid ether oil.

14. The cleansing composition according to any one of claims 1 to 3, wherein a content ratio of the aqueous layer and the oil layer is 10:90 to 90:10 in a mass ratio.

15. The cleansing composition according to any one of claims 1 to 3, wherein the viscosity at 25°C is not more than 100 mPa·s.

16. A two-layer separated cleansing composition consisting of an aqueous layer comprising (a) N-acyl acidic amino acid or a salt thereof, (b) a cationic surfactant, and (c) an alcohol having not more than 6 carbon atoms, and an oil layer comprising (e) a liquid oil agent, wherein the (a) N-acyl acidic amino acid or a salt thereof comprises (a1) a mixture of N-acyl acidic amino acid having an acyl group having 8 to 18 carbon atoms or a salt thereof, the (c) alcohol having not more than 6 carbon atoms comprises (c2) polyhydric alcohol having not more than 6 carbon atoms, and the aqueous layer comprises (f) N-acyl neutral amino acid having an acyl group having 8 to 18 carbon atoms or a salt thereof.

17. The cleansing composition according to claim 16, wherein the (a1) mixture of the N-acyl acidic amino acid having an acyl group having 8 to 18 carbon atoms or a salt thereof is a mixture of the N-acyl glutamic acid having an acyl group having 8 to 18 carbon atoms or a salt thereof.

18. The cleansing composition according to claim 16, wherein the (b) cationic surfactant is an alkyl ester of N-acyl basic amino acid or an acid addition salt thereof.

19. The cleansing composition according to claim 18, wherein the alkyl ester of N-acyl basic amino acid or an acid addition salt thereof is an alkyl ester having 1 to 3 carbon atoms of N-acyl basic amino acid having an acyl group having 8 to 18 carbon atoms or an acid addition salt thereof.

20. The cleansing composition according to claim 16, wherein the (c2) polyhydric alcohol having not more than 6 carbon atoms is at least one kind selected from the group consisting of 1,3-butyleneglycol, glycerol, and sorbitol.

21. The cleansing composition according to claim 16, wherein the aqueous layer further comprises (d) basic amino acid or a salt thereof.

22. The cleansing composition according to claim 21, wherein the (d) basic amino acid or a salt thereof is arginine or a salt thereof.

23. The cleansing composition according to claim 16, wherein the (e) liquid oil agent is at least one kind selected from the group consisting of light isoparaffin and liquid ether oil.

24. The cleansing composition according to claim 16, wherein the (f) N-acyl neutral amino acid having an acyl group having 8 to 18 carbon atoms or a salt thereof is N-acyl glycine having an acyl group having 8 to 18 carbon atoms or a salt thereof.

25. The cleansing composition according to any one of claims 16 to 24, wherein a content ratio of the aqueous layer and the oil layer is 10:90 to 90:10 in a mass ratio.

26. The cleansing composition according to any one of claims 16 to 24, wherein the viscosity at 25°C is not more than 100 mPa·s.
